# EUROPEAN PATENT APPLICATION

(11) **EP 4 098 748 A1**
(43) Date of publication of application: **07.12.2022**
(21) Application number: 20916937.4
(22) Date of filing: 25.12.2020
(51) Int. Cl.: C12N 15/13, C12N 1/20, C12N 1/21, C12N 15/31, C12P 21/08

(54) **METHOD FOR PRODUCING HEAVY CHAIN VARIABLE DOMAIN OF HEAVY-CHAIN ANTIBODY**

(30) Priority: 27.01.2020 JP 2020011149
(71) Applicant: Kao Corporation, Chuo-ku Tokyo 103-8210 (JP)
(72) Inventor: NISHIGUCHI, Hiroki, Wakayama-shi, Wakayama 640-8580 (JP); WADA, Mayumi, Wakayama-shi, Wakayama 640-8580 (JP); TOJO, Takuto, Wakayama-shi, Wakayama 640-8580 (JP); INOUE, Hidetoshi, Tokyo 131-8501 (JP); KAGEYAMA, Yasushi, Wakayama-shi, Wakayama 640-8580 (JP)
(74) Representative: Vossius & Partner Patentanwälte Rechtsanwälte mbB
(86) International application number: PCT/JP2020/048719
(87) International publication number: WO 2021/153129

(57) **Abstract**

Provided is a method for producing a heavy chain variable domain of a heavy-chain antibody (VHH) with a high yield. The method for producing a VHH includes culturing a *Bacillus* bacterium into which a gene that encodes variable domain of heavy chain of heavy-chain antibody is introduced and which is deficient in an extracellular protease.

## Description

### Field of the Invention

The present invention relates to a method for producing a variable domain of heavy chain of heavy-chain antibody.

### Background of the Invention

Small molecule antibodies have advantages, such as production efficiency, ease of drug design, and tissue permeability, and are therefore attracting attention as materials for antibody drugs and reagents. Examples of typical small molecule antibodies include a Fab, a single-chain variable fragment (single chain antibody; scFv), a diabody, and a variable domain of heavy chain of heavy-chain antibody (VHH). Sera of Camelidae animals (such as a Bactrian camel, an Arabian camel, and a llama) and Cartilaginous fish, such as a shark, contain antibodies lacking light chains and consisting only of heavy chains (heavy-chain antibodies). The variable domain of heavy-chain antibody is a VHH. The VHH is a molecule that can recognize an antigen through a single domain and is the smallest unit among antibody molecules that have been found to date. Pharmaceutical VHHs each including a plurality of domains or a humanized domain are also on sale.

Conventionally, *Escherichia coli* has been mainly used for microbiological production of antibodies. However, heterologous protein production by *Escherichia coli* has problems, such as low productivity of high molecular weight molecules, contamination of the product with endotoxin, and a reduction in yield of the product due to the treatment for removing the endotoxin. Non Patent Literature 1 describes that *Brevibacillus* can overcome the above-mentioned disadvantages of *Escherichia coli* and can produce an scFv, a Fab, or a VHH by a *Brevibacillus* expression system. Patent Literature 1 describes that there is a report that a VHH was produced using a Filamentous fungus such as *Aspergillus.*

*Bacillus* bacteria, such as *Bacillus subtilis,* secrete extracellular proteases, such as AprE, Epr, WprA, Mpr, NprB, Bpr, NprE, Vpr, and AprX. These extracellular proteases are known as factors that reduce the productivity of a target protein in heterologous protein production using *Bacillus* bacteria. Patent Literature 2 describes that the productivity of a heterologous protein is improved in a *Bacillus* strain deficient in extracellular proteases such as AprX. Non Patent Literature 2 describes secretary production of an scFv derived from a fibrin-specific monoclonal antibody in a *Bacillus subtilis* strain in which intracellular and extracellular molecular chaperones are co-expressed and the cell wall-binding protease WprA is inactivated. At the same time, Non Patent Literature 2 also describes that a *Bacillus subtilis* strain deficient in seven extracellular proteases could not produce the scFv.

(Patent Literature 1) JP-A-2012-157315
(Patent Literature 2) JP-A-2006-174707
(Non Patent Literature 1) Biotechnology, 2017, 95(11): 649-651
(Non Patent Literature 2) Appl. Environ. Microbiol., 2002, p. 3261-3269

### Summary of the Invention

The present invention provides a method for producing a variable domain of heavy chain of heavy-chain antibody, including culturing a *Bacillus* bacterium into which a gene that encodes variable domain of heavy chain of heavy-chain antibody is introduced and which is deficient in an extracellular protease.

The present invention provides a *Bacillus* bacterium into which a gene that encodes variable domain of heavy chain of heavy-chain antibody is introduced and which is deficient in an extracellular protease.

### Brief Description of Drawings

[Figure 1] Figure 1 shows SDS-PAGE images of culture supernatants from extracellular protease single deficient recombinant *Bacillus subtilis* strains incorporating VHH genes. The arrow indicates the band positions of VHH proteins.
[Figure 2] Figure 2 shows expression of pharmaceutical VHH molecules by a protease multiply deficient strain Dpr9ΔsigF.
[Figure 3] Figure 3 shows Western blotting images of Ozoralizumab and Caplacizumab expressed in Dpr9ΔsigF, where * indicates the full length, and < indicates degradation products.
[Figure 4] Figure 4 shows antigen (HEWL) binding activities of the VHH (1ZVH) expressed by *Bacillus subtilis.*
[Figure 5] Figure 5 shows Western blotting images of cultures of *Escherichia coli* strains incorporating VHH genes.

### Detailed Description of the Invention

As used herein, the term "antibody" refers to a molecule that can specifically bind to a target, such as a protein, via an antigen recognition domain derived from immunoglobulin.

As used herein, the term "VHH" refers to a variable domain of heavy chain of heavy-chain antibody. The heavy-chain antibody has been found in, for example, a Camelidae animal and Cartilaginous fish such as a shark. The VHH as used herein is derived from, for example, a Camelidae animal or a shark and is preferably derived from a Camelidae animal. The VHH is a molecule that can recognize an antigen through a single domain and is the smallest unit among antibody molecules that have been found to date. The VHH that is produced by the present invention can include one or more variable domains of heavy chain derived from a heavy-chain antibody, and the number of the variable domains of heavy chain included in the VHH is not limited. The variable domain of heavy chain included in the VHH produced by the present invention may be a variable domain heavy chain derived from a natural heavy-chain antibody or may be a variant thereof. The VHH produced by the present invention may include a fragment other than the variable domain of heavy chain derived from an immunoglobulin heavy chain (for example, a fragment of the constant region of the heavy-chain antibody, a human-derived fragment or a linker sequence) or amino acid mutation for stabilization. The VHH produced by the present invention does not include a variable domain derived from an immunoglobulin light chain as an antigen recognition domain. For example, a single-chain variable fragment including a variable domain of immunoglobulin light chain (e.g., scFv) is not encompassed in the VHH produced by the present invention.

Examples of the Camelidae animal as used herein include a Bactrian camel, an Arabian camel, a llama, an alpaca, a vicuna, and a guanaco, and an alpaca is preferable.

The identity of nucleotide sequences and amino acid sequences as described herein is calculated by the Lipman-Pearson method (Science, 1985, 227: 1435-1441). Specifically, the identity is calculated by analysis using the homology analysis (Search homology) program of genetic information processing software Genetyx-Win and setting the Unit size to compare (ktup) to 2.

As used herein, the term "identity of at least 85%" regarding an amino acid sequence or a nucleotide sequence refers to an identity of 85% or more, preferably 90% or more, more preferably 93% or more, further preferably 95% or more, further preferably 96% or more, further preferably 97% or more, further preferably 98% or more, further preferably 99% or more, and further preferably 99.5% or more.

As used herein, the term "operable linkage" between a control region and a gene refers that a gene and a control region are linked to each other such that the gene can be expressed under the control of the control region. The procedure of the "operable linkage" between a gene and a control region is well known to those skilled in the art.

As used herein, the terms of "upstream" and "downstream" regarding a gene refer to the upstream and the downstream, respectively, in the transcriptional direction of the gene. For example, "a gene located downstream a promoter" means that the gene is present on the 3' side of the promoter in the DNA sense strand, and the upstream a gene means the region on the 5' side of the gene in the DNA sense strand.

As used herein, a "deficiency" of a protease in a cell means that the activity of the protease in the cell is decreased compared to the original activity in the cell (non-deficient strain, such as a wild strain). The "deficiency" of a protease includes not only a decrease or loss in protease protein expression but also a decrease or loss in protease protein function. A cell deficient in a protease (protease deficient strain) can be produced by partial or complete deletion or inactivation (e.g., so-called knockdown or knockout) of the protease gene in the cell. Preferably, in the protease deficient strain as described herein, the protease activity is decreased to 50% or less, more preferably 25% or less, with respect to the wild strain.

As used herein, the term "original" used for the function, property, and trait of a cell is used to refer to the function, property, and trait that are naturally present in the cell. In contrast, the term "foreign" is used to refer to the function, property, and trait that are not originally present in the cell but are introduced exogenously. For example, a "foreign" gene or polynucleotide is a gene or polynucleotide introduced exogenously into a cell. A foreign gene or polynucleotide may be derived from an organism of the same species as that of the cell into which it was introduced or may be derived from an organism of a different species (i.e., a heterologous gene or polynucleotide).

As used herein, *"Bacillus* bacterium" refers to a bacterium belonging to the genus *Bacillus* of the family Bacillaceae. Examples of *Bacillus* bacterium include *Bacillus subtilis, Bacillus cereus, Bacillus thuringiensis, Bacillus megaterium, Bacillus amyloliquefaciens, Bacillus pumilus,* and *Bacillus liqueniformis.* Bacteria belonging to the genus *Paenibacillus* or *Brevibacillus* of the family Paenibacillaceae are not included in the *Bacillus* bacteria as used herein.

The names of genes of *Bacillus subtilis* as described herein are based on *Bacillus subtilis* genomic data published on the internet by JAFAN: Japan Functional Analysis Network for *Bacillus subtilis* (BSORF DB) (bacillus.genome.ad.jp/, renewed on January 18, 2006). The gene numbers of *Bacillus subtilis* as described herein are gene numbers registered in the BSORF DB.

The present invention provides a method for producing a variable domain of heavy chain of heavy-chain antibody (VHH).

The present inventors found that the VHH can be produced with a high yield by *Bacillus* bacteria deficient in an extracellular protease.

According to the present invention, the VHH can be produced with a high yield. The present invention can overcome problems in conventional antibody production by *Escherichia coli,* such as a low yield and contamination with endotoxin. Since *Bacillus* bacteria have many use results in the production of useful substances, such as enzymes, and are not harmful, the method of the present invention is easy and safe to operate.

The present invention provides a method for producing a VHH, including culturing a *Bacillus* bacterium into which a gene that encodes the VHH is introduced and which is deficient in an extracellular protease.

The VHH produced by the present invention is as defined above. More specific examples of the VHH produced by the present invention include polypeptides consisting of the amino acid sequences of SEQ ID NOs: 2, 4, 6, 8, 10, 12, 14, 16, 18, 20, 22, 24, 26, 28, 30, 32, 34, 36, and 38. Detailed information on these VHHs are as shown in Table 1.

**[Table 1]**

| **SEQ ID:No** | **Name** | **Reference** | **Gene ID** |
|---|---|---|---|
| **2** | **3G9A** | **Protein data bank** | **3G9A** |
| **4** | **4C57** | **Protein data bank** | **4C57** |
| **6** | **5IMM** | **Protein data bank** | **5IMM** |
| **8** | **5JQH** | **Protein data bank** | **5JQH** |
| **10** | **5NBD** | **Protein data bank** | **5NBD** |
| **12** | **3K1K** | **Protein data bank** | **3K1K** |
| **14** | **4FHB** | **Protein data bank** | **4FHB** |
| **16** | **4KSD** | **Protein data bank** | **4KSD** |
| **18** | **3K81** | **Protein data bank** | **3K81** |
| **20** | **5FV2** | **Protein data bank** | **5FV2** |
| **22** | **4W6W** | **Protein data bank** | **4W6W** |
| **24** | **5J57** | **Protein data bank** | **5J57** |
| **26** | **N15** | Mizukami, M. et al., Protein Expression and Purifi cation 105, 23-32 (2015**).** | |
| **28** | **4GFT** | **Protein data bank** | **4GFT** |
| **30** | **Ozoralizumab** | **G-SRS** | **05ZCK72TXZ** |
| **32** | **Caplacizumab** | **G-SRS** | **2R27AB6766** |
| **34** | **NbHuI6_VGLW** | Vincke, C. et al., J. Biol. Chem., 284, 3273-3284 (2009**).** | |
| **36** | **h-NbBcII10_FGLA** | Vincke, C. et al., J. Biol. Chem., 284, 3273-3284 (2009**).** | |
| **38** | **1ZVH** | **Protein data bank** | **1ZVH** |

Other examples of the VHH produced by the present invention include polypeptides consisting of amino acid sequences having an identity of at least 85% to any one of the amino acid sequences of SEQ ID NOs: 2, 4, 6, 8, 10, 12, 14, 16, 18, 20, 22, 24, 26, 28, 30, 32, 34, 36, and 38.

Further other examples of the VHH produced by the present invention include polypeptides consisting of the amino acid sequences of SEQ ID NOs: 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, and 138. Further other examples of the VHH produced by the present invention include polypeptides consisting of amino acid sequences having an identity of at least 85% to any one of the amino acid sequences of SEQ ID NOs: 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, and 138.

A gene (polynucleotide) encoding the VHH can be prepared in accordance with a usual method. Examples of the gene that encodes the VHH include polynucleotides consisting of the nucleotide sequences of SEQ ID NOs: 1, 3, 5, 7, 9, 11, 13, 15, 17, 19, 21, 23, 25, 27, 29, 31, 33, 35, 37, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, and 137, and polynucleotides having a sequence identity of at least 85% to any one of these sequences. For example, the polynucleotide of a gene that encodes the VHH can be chemically synthesized in accordance with sequence information. Alternatively, the polynucleotide of a gene that encodes the VHH can be separated from the genomic DNA of a Camelidae animal. The resulting gene may be further subjected to mutagenesis.

The gene that encodes the VHH may be operably linked to a control region. As used herein, the "control region" is a region that has a function of controlling expression of a gene located downstream the region in a cell, preferably a region that has a function of constitutively or highly expressing the gene located downstream. More specifically, the control region can be defined as a region present upstream the coding region of the gene and having a function of controlling the transcription of the coding region through an interaction with an RNA polymerase. Preferably, the control region as used herein refers to a region of about 200 to 600 nucleotides upstream the coding region of the gene. The control region includes the transcription initiation control region and/or the translation initiation control region, or a region from the transcription initiation control region to the translation initiation control region. The transcription initiation control region is a region including a promoter and a transcription initiation point, and the translation initiation control region is a site equivalent to a Shine-Dalgarno (SD) sequence that forms a liposome binding site together with an initiation codon (Proc. Natl. Acad. Sci. USA, 1974, 71: 1342-1346).

Preferable examples of the control region include control regions that function in *Bacillus* bacteria, for example, the control regions of α-amylase gene, protease gene, aprE gene, and spoVG gene derived from *Bacillus* bacteria, the control region of a cellulase gene of *Bacillus* sp. KSM-S237 strain (JP-A-2000-210081), the control region of a cellulase gene of *Bacillus* sp. KSM-64 strain (JP-A-2011-10387), and the control regions of a kanamycin resistance gene and a chloramphenicol resistance gene derived from *Staphylococcus aureus* (see JP-A-2009-089708 for both), but the control region is not particularly limited. More preferable examples of the control region include the promoter (SEQ ID NO: 39) of the cellulase gene of *Bacillus* sp. KSM-S237 strain, the promoter (SEQ ID NO: 40) of the cellulase gene of *Bacillus* sp. KSM-64 strain, and the promoter (SEQ ID NO: 41) of the *Bacillus subtilis* spoVG gene. Preferable examples of the control region include polynucleotides having an identity of at least 85% to any one of SEQ ID NOs: 39 to 41 and having a function of controlling transcription and translation of the gene.

The gene that encodes the VHH may be operably linked to a sequence (referred to as secretion signal sequence) encoding a secretion signal having a function of secreting the expressed protein to the outside of the cell. Preferable examples of the secretion signal sequence include secretion signal sequences that function in *Bacillus* bacteria, for example, a secretion signal sequence derived from a *Bacillus* bacterium. Preferable examples of the secretion signal sequence derived from a *Bacillus* bacterium include a secretion signal sequence (SEQ ID NO: 42) of a cellulase gene of *Bacillus* sp. KSM-S237 strain, a secretion signal sequence (SEQ ID NO: 43) of a cellulase gene of *Bacillus* sp. KSM-64 strain, and a secretion signal sequence (SEQ ID NO: 44) of *Bacillus subtilis* amylase gene amyE. Furthermore, examples of the secretion signal sequence derived from a *Bacillus* bacterium include polynucleotides having an identity of at least 85% to any one of SEQ ID NOs: 42 to 44 and having a function of secreting the expressed protein to the outside of the cell.

The gene that encodes the VHH may include a nucleotide sequence of an untranslated region (UTR), in addition to an open reading frame (ORF). For example, the gene may include the above-described promoter and secretion signal sequence and a terminator.

The gene can be introduced into a *Bacillus* bacterium in accordance with a usual method. For example, the gene can be incorporated into the genome of a *Bacillus* host cell by introducing the gene or a vector including the gene into the host cell. Alternatively, an expression vector including the gene may be introduced into a *Bacillus* host cell.

A gene or a vector can be introduced into a *Bacillus* host cell by, for example, using a known transformation technique, such as a competent cell method, an electroporation method, a protoplast method, a particle gun method, or a PEG method.

Examples of the *Bacillus* host cell into which a gene that encodes the VHH is to be introduced include the above-mentioned bacteria of the genus *Bacillus* of the family Bacillaceae, and among these bacteria, *Bacillus subtilis* and a mutant strain thereof are preferable.

A vector including a gene that encodes the VHH can be constructed by inserting a polynucleotide including the gene and as needed, a control region or a secretion signal sequence into an arbitrary vector by a usual method and linking them. The type of the vector is not particularly limited, and the vector may be an arbitrary vector, such as a plasmid, a phage, a phagemid, a cosmid, a virus, a YAC vector, or a shuttle vector. The vector is preferably a vector that can amplify in the host cell and more preferably an expression vector. Preferable examples of the vector include, but not limited to, shuttle vectors, such as pHA3040SP64, pHSP64R, and pASP64 (Japanese Patent No. 3492935), pHY300PLK (an expression vector capable of transforming both *E*scherichia coli and Bacillus subtilis, Jpn. J. Genet., 1985, 60: 235-243), and pAC3 (Nucleic Acids Res., 1988, 16: 8732); and plasmids that can be used for transformation of *Bacillus* bacteria, such as pUB110 (J. Bacteriol., 1978, 134: 318-329) and pTA10607 (Plasmid, 1987, 18: 8-15). Plasmids derived from *Escherichia coli* (for example, pET22b(+), pBR322, pBR325, pUC57, pUC118, pUC119, pUC18, pUC19, and pBluescript) can also be used.

The *Bacillus* bacterium used for producing a VHH in the present invention has a gene that encodes the VHH introduced thereinto and also is deficient in an extracellular protease. The extracellular protease that the *Bacillus bacterium* is deficient in is preferably at least one selected from the group consisting of extracellular proteases encoded by epr, wprA, bpr, vpr, aprE, aprX, and genes equivalent to these genes, more preferably at least one selected from the group consisting of proteases encoded by epr, wprA, bpr, vpr, aprE, and apr, further preferably a protease encoded by epr, wprA, bpr, vpr, aprE, or apr, and further preferably a protease encoded by wprA or bpr.

The genes, epr, wprA, bpr, vpr, aprE, and aprX, are all *Bacillus subtilis* genes. The gene numbers of these genes and the functions of proteins encoded by these genes are shown in Table 2. Examples of the gene equivalent to epr, wprA, bpr, vpr, aprE, or aprX include genes derived from *Bacillus* bacteria and having a nucleotide sequence having an identity of at least 85% to epr, wprA, bpr, vpr, aprE, or aprX and encoding proteins with the same functions (as those shown in Table 2). These genes can be searched in the above-mentioned BSORF DB.

**[Table 2]**

| **Gene name** | **BSORF DB Gene No.** | **Function of encoded protein** |
|---|---|---|
| **epr** | **BG10561** | **Minor extracellular serine protease** |
| **wprA** | **BG11846** | **Cell wall binding protease precursor (CWBP23, CWBP52)** |
| **bpr** | **BG10233** | **Bacillopeptidase F** |
| **vpr** | **BG10591** | **Minor extracellular serine protease** |
| **aprE** | **BG10190** | **Serine alkaline protease (subtilisin E)** |
| **aprX** | **BG12567** | **Intracellular serine protease (secreted to the outside of cell)** |

The *Bacillus* bacterium that is used in the present invention may be deficient in an extracellular protease other than the extracellular proteases encoded by above-mentioned epr, wprA, bpr, vpr, aprE, or aprX. For example, the *Bacillus* bacterium that is used in the present invention may be deficient in all extracellular proteases encoded by epr, wprA, mpr, nprB, bpr, nprE, vpr, aprE, or aprX. The *Bacillus* bacterium that is used in the present invention may have a mutation other than the extracellular protease deficiency, for example, deletion of a sigma factor such as sigF.

A *Bacillus* bacterium deficient in an extracellular protease can be produced in accordance with a usual method. For example, a *Bacillus* bacterium deficient in an extracellular protease can be produced by deleting or inactivating the gene that encodes the extracellular protease in the genomic DNA of the bacterium. Accordingly, in an embodiment of the present invention, the *Bacillus* bacterium that is used for producing a VHH includes a deletion or inactivation of at least one gene selected from the group consisting of epr or a gene equivalent thereto, wprA or a gene equivalent thereto, bpr or a gene equivalent thereto, vpr or a gene equivalent thereto, aprE or a gene equivalent thereto, and aprX or a gene equivalent thereto. The *Bacillus* bacterium preferably includes a deletion or inactivation of at least one gene selected from the group consisting of epr, wprA, bpr, vpr, aprE, and aprX. The *Bacillus* bacterium more preferably includes a deletion or inactivation of epr, wprA, bpr, vpr, aprE, or aprX. The *Bacillus* bacterium further preferably includes a deletion or inactivation of wprA or bpr.

Examples of the method for deleting or inactivating a gene include mutation introduction to the gene or the structural region thereof, for example, substitution or insertion of one or more nucleotides into the polynucleotide of the gene or its structural region or deletion of a part or the whole of the sequence; inactivation of a promoter by substitution or insertion of another sequence into the promoter region of the gene; and introduction of an anti-sense nucleic acid into the gene. Examples of specific method for mutation introduction include UV irradiation, site-directed mutagenesis, an SOE-PCR method (splicing by overlap extension PCR: Gene, 1989, 77: 61-68), and a homologous recombination method. The position on the genomic DNA and the nucleotide sequence of a gene to be deleted or inactivated can be confirmed with the above-mentioned BSORF DB.

In the present invention, a recombinant *Bacillus* bacterium into which a polynucleotide that encodes the VHH prepared by the above-described procedure is introduced and which is deficient in an extracellular protease is used. In the procedure of preparation of the recombinant *Bacillus* bacterium, the order of the introduction of the polynucleotide that encodes a VHH and the protease deficiency treatment is not particularly limited as long as the target recombinant *Bacillus* bacterium is obtained.

In the present invention, a target VHH is produced by culturing the above-described recombinant *Bacillus* bacterium into which a polynucleotide that encodes the VHH is introduced and which is deficient in an extracellular protease. The recombinant *Bacillus* bacterium may be cultured in accordance with a usual method for culturing a *Bacillus* bacterium. For example, a medium for culturing the *Bacillus* bacterium includes a carbon source and nitrogen source necessary for growth of the bacterium. Examples of the carbon source include glucose, dextran, soluble starch, sucrose, and methanol. Examples of the nitrogen source include ammonium salts, nitrates, amino acid, corn steep liquor, peptone, casein, meat extract, soybean cake, and potato extraction liquid. The medium may include, as needed, other nutrients, such as an inorganic salt (e.g., sodium chloride, calcium chloride, sodium dihydrogen phosphate, and magnesium chloride), vitamins, and an antibiotic (e.g., tetracycline, neomycin, kanamycin, spectinomycin, and erythromycin). Culture conditions, such as temperature, aeration-agitation condition, pH of the medium, and culture time, can be appropriately selected depending on the bacterial species, trait, culture scale, etc.

A VHH is expressed in cells by culturing the recombinant *Bacillus* bacterium. Furthermore, when the gene of the VHH is linked to a secretion signal, the expressed VHH is secreted outside the cells. The VHH produced by the method of the present invention can be collected from the culture solution by using a usual method that is used in protein purification, for example, by using cell disruption, centrifugation, ammonium sulfate precipitation, gel chromatography, ion exchange chromatography, or affinity chromatography alone or in appropriate combination thereof.

The present invention also encompasses, as exemplary embodiments, the following substances, production methods, uses, methods, etc. However, the present invention is not limited to these embodiments.
[1] A method for producing a variable domain of heavy chain of heavy-chain antibody, comprising culturing a *Bacillus* bacterium into which a gene that encodes variable domain of heavy chain of heavy-chain antibody is introduced and which is deficient in an extracellular protease.
[2] The method according to [1], wherein the extracellular protease is:
   preferably at least one selected from the group consisting of extracellular proteases encoded by epr, wprA, bpr, vpr, aprE, aprX, and genes equivalent to these genes;
   more preferably an extracellular protease encoded by epr, wprA, bpr, vpr, aprE, or aprX; and
   further preferably an extracellular protease encoded by wprA or bpr.
[3] The method according to [1] or [2], wherein the variable domain of heavy chain of heavy-chain antibody preferably consists of an amino acid sequence of SEQ ID NO: 2, 4, 6, 8, 10, 12, 14, 16, 18, 20, 22, 24, 26, 28, 30, 32, 34, 36, 38, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, or 138 or an amino acid sequence having an identity of at least 85% to any of these sequences.
[4] The method according to any one of [1] to [3], wherein the *Bacillus* bacterium is preferably *Bacillus subtilis* or a mutant strain thereof.
[5] The method according to any one of [1] to [4], wherein the *Bacillus* bacterium deficient in an extracellular protease is preferably a *Bacillus* bacterium in which the gene that encodes the extracellular protease is deleted or inactivated.
[6] A *Bacillus* bacterium into which a gene that encodes variable domain of heavy chain of heavy-chain antibody is introduced and which is deficient in an extracellular protease.
[7] The *Bacillus* bacterium according to [6], wherein the extracellular protease is:
   preferably at least one selected from the group consisting of extracellular proteases encoded by epr, wprA, bpr, vpr, aprE, aprX, and genes equivalent to these genes;
   more preferably an extracellular protease encoded by epr, wprA, bpr, vpr, aprE, or aprX; and
   further preferably an extracellular protease encoded by wprA or bpr.
[8] The *Bacillus* bacterium according to [6] or [7], wherein the variable domain of heavy chain of heavy-chain antibody preferably consists of an amino acid sequence of SEQ ID NO: 2, 4, 6, 8, 10, 12, 14, 16, 18, 20, 22, 24, 26, 28, 30, 32, 34, 36, 38, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, or 138 or an amino acid sequence having an identity of at least 85% to any of these sequences.
[9] The *Bacillus* bacterium according to any one of [6] to [8], which is preferably *Bacillus subtilis* or a mutant strain thereof.
[10] The *Bacillus* bacterium according to any one of [6] to [9], wherein the gene that encodes the extracellular protease is preferably deleted or inactivated.

### Examples

The present invention will now be more specifically described using examples. However, the present invention is not limited to these examples.

### Materials

### (1) Strain

Strains deficient in extracellular protease genes (epr, wprA, mpr, nprB, bpr, nprE, vpr, aprE, and aprX) were produced from a *Bacillus subtilis* 168 strain (hereinafter, refers to as 168 strain) in accordance with the method described in Patent Literature 2. Hereinafter, the resulting extracellular protease single deficient strains are respectively referred to as Δepr, Δ wprA, Δmpr, ΔnprB, Δbpr, ΔnprE, Δvpr, ΔaprE, and ΔaprX. The sigF gene involved in spore formation was deleted from a *Bacillus subtilis* strain Dpr9 deficient in all above-mentioned nine extracellular protease genes in accordance with the method described in JP-B-4336082. The resulting extracellular protease multiply deficient strain is referred to as Dpr9ΔsigF.

As *Escherichia coli,* Rosetta-gami 2(DE3)pLysS strain (Novagen) was used.

### (2) Medium

LB medium: 1% Bacto^{™} Tryptone, 0.5% Bacto^{™} Yeast Extract, and 1% sodium chloride. A plate medium including 1.5% agar and as needed, tetracycline (50 ppm).

DM3 medium: 1% CMC (Kanto Chemical Co., Inc.), 0.5% Bacto^{™} Casamino Acids, 0.5% Bacto^{™} Yeast Extract, 8.1% disodium succinate-6H₂O, 0.35% dipotassium hydrogen phosphate, 0.15% potassium dihydrogen phosphate, 0.5% glucose, 20 mM magnesium chloride, 0.01% BSA, and 50 ppm tetracycline. A plate medium including 1% agar.

2× L-mal medium: 2% Bacto^{™} Tryptone, 1% Bacto^{™} Yeast Extract, 1% sodium chloride, 7.5% maltose monohydrate, 7.5 ppm manganese sulfate, and 15 ppm tetracycline.

### (3) Reagent

Reagents were those produced by FUJIFILM Wako Pure Chemical Corporation unless otherwise stated.

### Example 1 VHH production by protease deficient recombinant Bacillus subtilis

### (1) Artificial synthesis of gene

VHH genes (polynucleotides of SEQ ID NOs: 1, 3, 5, 7, 9, 11, 13, 15, 17, 19, 21, 23, 25, 27, 29, 31, 33, 35, and 37 encoding VHHs of SEQ ID NOs: 2, 4, 6, 8, 10, 12, 14, 16, 18, 20, 22, 24, 26, 28, 30, 32, 34, 36, and 38, respectively) were artificially synthesized by Thermo Fisher Scientific. GCAGCTCTTGCAGCA (SEQ ID NO: 45) and TCTATTAAACTAGTT (SEQ ID NO: 46) were added to the 5' terminal and the 3' terminal, respectively, of each of the artificially synthesized genes as sequences for inserting the gene into a plasmid vector.

### (2) Construction of plasmid for VHH expression

A plasmid sequence was amplified by PCR using recombinant plasmid pHY-S237 (JP-A-2014-158430) as a template and using a primer set of SEQ ID NO: 47 (5'-TGCTGCAAGAGCTGCCGGAAATAAA-3') and SEQ ID NO: 48 (5'-TCTATTAAACTAGTTATAGGG-3') and PrimeSTAR Max DNA polymerase (TAKARA BIO INC.). DNAs including the artificially synthesized genes of (1) were incorporated into the resulting PCR fragment using In-Fusion HD Cloning Kit (TAKARA BIO INC.) to construct VHH expression plasmids including the artificially synthesized VHH genes shown in (1), respectively. The constructed plasmids were introduced into Dpr9ΔsigF in accordance with the procedure shown in the following (3).

In the gene that encodes the VHH of SEQ ID NO: 26, the promoter region was changed to that derived from a *Bacillus subtilis* spoVG gene by the following procedure. A plasmid sequence was amplified by PCR using a VHH expression plasmid including the gene of SEQ ID NO: 25 as a template and using a primer set of SEQ ID NO: 49 (5'-GATCCCCGGGAATTCCTGTTATAAAAAAAGG-3') and SEQ ID NO: 50 (5'-ATGATGTTAAGAAAGAAAACAAAGCAG-3') and PrimeSTAR Max DNA polymerase (TAKARA BIO INC.). A promoter DNA derived from the spoVG gene was amplified by PCR using the genome of the 168 strain as a template and using a primer set of SEQ ID NO: 51 (5'-GAATTCCCGGGGATCTAAGAAAAGTGATTCTGGGAGAG-3') and SEQ ID NO: 52 (5'-CTTTCTTAACATCATAGTAGTTCACCACCTTTTCCC-3'). The resulting promoter DNA was incorporated into the plasmid sequence using In-Fusion HD Cloning Kit (TAKARA BIO INC.) to construct a VHH expression plasmid including the VHH gene of SEQ ID NO: 25 linked to the spoVG promoter. The constructed plasmid was introduced into each of the protease single deficient strains Δepr, ΔwprA, Δmpr, ΔnprB, Δbpr, ΔnprE, Δvpr, ΔaprE, and ΔaprX in accordance with the procedure shown in the following (3).

### (3) Production of recombinant Bacillus subtilis

Plasmid introduction into the *Bacillus subtilis* strain was performed by a protoplast method shown below. Glycerol stock of each type of *Bacillus subtilis* was inoculated in 1 mL of an LB liquid medium, followed by shake culture at 210 rpm overnight at 30°C. On the following day, 10 µL of this culture solution was inoculated in 1 mL of a new LB liquid medium, followed by shake culture at 210 rpm for about 2 hours at 37°C. This culture solution was collected in a 1.5-mL tube and was centrifuged at 12,000 rpm for 5 minutes, and the pellet obtained by removing the supernatant was suspended in 500 µL of SMMP including 4 mg/mL of Lysozyme (Sigma-Aldrich Co., LLC), followed by incubation at 37°C for 1 hour. Subsequently, centrifugation was performed at 3,500 rpm for 10 minutes, and the pellet obtained by removing the supernatant was suspended in 400 µL of SMMP. Thirty-three microliters of this suspension was mixed with each plasmid, and 100 µL of 40% PEG was further added to each mixture, followed by vortexing. To this solution, 350 µL of SMMP was added, followed by mixing with inversion and shaking at 30°C at 210 rpm for 1 hour. The whole resulting mixture was then applied to a DM3 agar medium plate and was incubated at 30°C for 2 to 3 days.

### (4) VHH production

The recombinant *Bacillus subtilis* produced in (3) was inoculated in 1 mL of an LB medium containing 50 ppm tetracycline, followed by reciprocal shaking at 32°C overnight to obtain a pre-culture solution. For the protease single deficient strains, the pre-culture solution was each inoculated at 1% in 5 mL of a 2× L-mal medium in a test tube, followed by shake culture at 30°C for 72 hours. For Dpr9ΔsigF, the pre-culture solution was inoculated at 1% in 20 mL of a 2× L-mal medium in a baffled Erlenmeyer flask, followed by shake culturing at 30°C for 72 hours. At the end of the culturing, 1 mL of the culture solution was centrifuged in a microtube at 4°C at 15,000 rpm for 5 minutes, and the supernatant was collected.

### (5) SDS-PAGE

The culture supernatant obtained in (4) and a 50 mM DTT-containing Laemmli Sample Buffer (Bio-Rad Laboratories, Inc.) were mixed in equivalent quantities and were then heat-treated at 99°C for 5 minutes to prepare a sample. Mini-PROTEIN TGX Stain-Free (Bio-Rad Laboratories, Inc.) was used as the gel. Five microliters of the sample was applied to each well and was then electrophoresed at 210 V for 25 minutes. Precision Plus protein Unstained standard (Bio-Rad Laboratories, Inc.) was used as the molecular weight marker. The protein bands were detected by ChemiDoc MP Imaging System. The detected protein was quantitatively measured using a calibration curve formed based on a lysozyme standard (Sigma-Aldrich Co., LLC). In the quantitative measurement of the protein, software Image Lab produced by Bio-Rad Laboratories, Inc was used.

### (6) Western blotting

The protein was transferred from the SDS-PAGE gel to a PVDF membrane using Trans-Blot Turbo Mini PVDF Transfer Packs (Bio-Rad Laboratories, Inc.) and Trans-Blot Turbo System (Bio-Rad Laboratories, Inc.). The antibody used was 6× -His Tag Monoclonal Antibody (3D5)-HRP (Invitrogen), and the antibody reaction used was iBind Western System (Invitrogen). The target protein was detected using 1-Step Ultra TMB-Blotting Solution (Thermo Fisher Scientific). The protein was quantitatively measured using a calibration curve formed based on concentration-known His-N15 purified protein (SEQ ID NO: 26). In the quantitative measurement of the protein, software Image Lab produced by Bio-Rad Laboratories, Inc. was used.

### (7) Intermolecular interaction measurement

The antigen-binding activity of the 1ZVH (SEQ ID NO: 38) produced by Dpr9ΔsigF was analyzed by intermolecular interaction measurement using Biacore T200 (GE Healthcare). The sensor chip used was Series S Sensor Chip CM5, and the running buffer used was HBS-EP+. HEWL (lysozyme, derived from egg albumen, FUJIFILM Wako Pure Chemical Corporation) was adjusted to 0.7 g/L with 25 mM Na phosphate pH 6.5 buffer to prepare an antigen solution. The antigen solution diluted 100-fold with an acetic acid aqueous solution (pH 4.0, 4.5, 5.0, or 5.5) to 7 µg/mL was used as substrates. As samples, 1ZVH diluted with the running buffer to 8.6 nM, 26 nM, 78 nM, 230 nM, or 700 nM was used. The following items were set or performed on the control software attached to Biacore T200 using the substrates and the samples, and the binding between the HEWL and the 1ZVH was monitored. Immobilisation pH scouting was performed to investigate the optimum pH for immobilization to a sensor chip. The optimum pH condition was judged to be pH 5.5. The target was set to 150 RU with Aim for immobilized level, and lysozyme was immobilized to Flow path 2 by an amine coupling method. In Flow path 1, Blank Immobilization was performed. The binding between a substrate and a sample was monitored in Flow path 2-1. The Regeneration Solution used was 10 mM Glycine-HCl (pH 1.5). In the binding monitoring, the contact time was 120 sec, the flow rate was 30 µL/min, the dissociation time was 120 sec, the contact time of regeneration was 30 sec, and the stabilization period was 120 sec. The kinetics were analyzed using the resulting sensor gram. The reaction model equation selected was 1:1 Binding.

### (8) Results

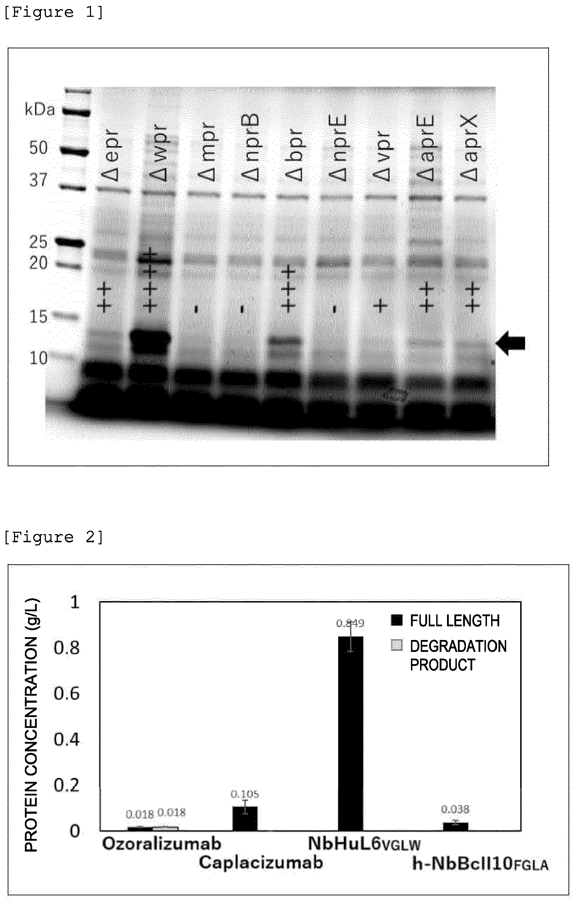

Figure 1 shows SDS-PAGE images of culture supernatants from extracellular protease single deficient recombinant *Bacillus subtilis* strains Δepr, ΔwprA, Δmpr, ΔnprB, Δbpr, ΔnprE, Δvpr, ΔaprE, and ΔaprX. In the figure, "+" indicates the strength of the band (indicated by the arrow in the figure) of an antibody. The VHH was highly expressed in Δepr, ΔwprA, Δbpr, Δvpr, ΔaprE, and ΔaprX. In particular, the VHH expressions in ΔwprA and Δbpr were significantly high. It was revealed from these results that deficiency of epr, wprA, bpr, vpr, aprE, or aprX affects the expression of the VHH in *Bacillus subtilis.*

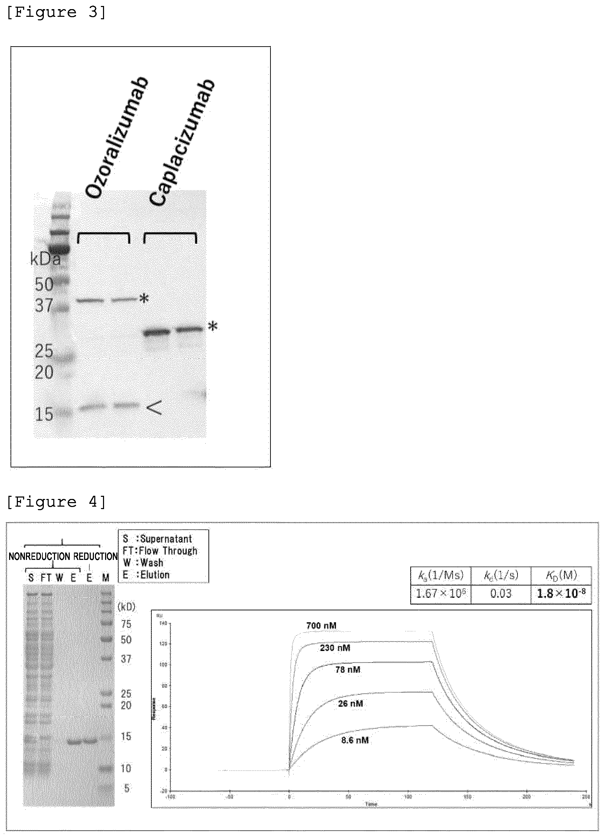

Table 3 shows the expression levels of the VHHs of SEQ ID NOs: 2, 4, 6, 8, 10, 12, 14, 16, 18, 20, 22, 24, 26, and 28 in the extracellular protease multiply deficient recombinant *Bacillus subtilis* strain Dpr9ΔsigF. The expressions of SEQ ID NO: 4 and SEQ ID NO: 28 were quantitatively measured by Western blotting, and the expressions of the remaining sequences were quantitatively measured by SDS-PAGE. Various VHHs could be produced by Dpr9ΔsigF. Figure 2 shows expression levels of pharmaceutical VHH molecules (Ozoralizumab and Caplacizumab) of SEQ ID NOs: 30 and 32 and pharmaceutical humanized VHH molecules (NbHul6_VGLW and h-NbBcII10_FGLA) of SEQ ID NOs: 34 and 36 in Dpr9ΔsigF. Figure 3 shows Western blotting images of Ozoralizumab and Caplacizumab expressed in Dpr9ΔsigF. Dpr9ΔsigF could express all four pharmaceutical VHH molecules at full length (see Figures 2 and 3). It was revealed from these results that the protease deficient *Bacillus subtilis* strain can express, for example, a VHH, a humanized VHH, and a molecule in which a plurality of VHHs are linked.

**[Table 3]**

| **VHH** | **SEQ ID:No** | **(g/L)** |
|---|---|---|
| **3G9A** | **2** | **0.31** |
| **4C57** | **4** | **0.3** |
| **5IMM** | **6** | **0.88** |
| **5JQH** | **8** | **0.41** |
| **5NBD** | **10** | **0.39** |
| **3k1k** | **12** | **0.51** |
| **4FHB** | **14** | **1.76** |
| **4KSD** | **16** | **0.11** |
| **3K81** | **18** | **0.86** |
| **5FV2** | **20** | **0.19** |
| **4W6W** | **22** | **0.16** |
| **5J57** | **24** | **0.12** |
| **N15** | **26** | **0.49** |
| **4GFT** | **28** | **0.02** |

Figure 4 shows electrophoresis images (left) of the culture supernatant of Dpr9ΔsigF and a fraction obtained during purification and antigen (HEWL) binding activity (right) of the purified VHH (1ZVH, SEQ ID NO: 38) measured in (7). In the electrophoresis, the Dpr9ΔsigF culture supernatant (S) was applied to a column filled with Ni-NTA Agarose (FUJIFILM Wako Pure Chemical Corporation) to obtain a fraction FT passed through the column. Subsequently, a washing buffer (30 mM imidazole, 0.25 M NaCl, 10 mM Tris pH 8.0) was applied to the column to obtain a fraction W passed through the column. An elution buffer (500 mM imidazole, 0.5 M NaCl, 20 mM Tris pH 7.5) was then applied to the column to obtain a fraction E passed through the column. The resulting S and fractions FT, W, and E were treated with Laemmli Sample Buffer (Bio-Rad Laboratories, Inc.) not containing DTT. The fraction E was treated with Laemmli Sample Buffer (Bio-Rad Laboratories, Inc.) containing 50 mM DTT. The treated samples were subjected to SDS-PAGE. The band shifted upward in the fraction E treated with the buffer containing DTT (reductive E) compared to the fraction E treated with the buffer not containing DTT (non-reductive E). This suggests that a disulfide bond is formed in the VHH expressed in *Bacillus subtilis.* As shown in the right of Figure 4, it was recognized by the intermolecular interaction measurement that the expressed VHH increased the level of response to the antigen immobilized on a concentration dependent sensor and therefore had an antigen-binding activity. It was revealed from this result that an activated VHH can be produced using *Bacillus subtilis.*

### Comparative Example 1 VHH production by recombinant Escherichia coli

### (1) Production of recombinant Escherichia coli

DNAs containing artificially synthesized genes (polynucleotides of SEQ ID NOs: 1, 3, 5, 7, and 9 encoding VHHs of SEQ ID NOs: 2, 4, 6, 8, and 10, respectively) shown in Example 1 (1) were amplified by PCR using the primers shown in Table 4 and PrimeSTAR Max DNA polymerase (TAKARA BIO INC.). pET22b was digested with Nco I and Hind III, and incorporation of the resulting PCR fragment was performed using In-Fusion HD Cloning Kit to construct a plasmid. The constructed plasmid was mixed with competent cells of Rosetta-gami 2(DE3)pLysS strain, left to stand on ice for 10 minutes, and then treated at 42°C for 1 minute to be introduced into an *Escherichia coli* strain.

**[Table 4]**

| **VHH** | **SEQ ID:No** | **Primer** | | **SEQ ID:No** |
|---|---|---|---|---|
| **3G9A** | **2** | **F** | **CCGGCGATGGCCATGGCAGATGTTCAACTGCAAGA** | **53** |
| | | **R** | **GTGCGGCCGCAAGCTTTAATGATGGTGGTGATGAT** | **54** |
| **4C57** | **4** | **F** | **CCGGCGATGGCCATGCAAGTCCAACTGCAAGAATC** | **55** |
| | | **R** | **GTGCGGCCGCAAGCTTTAATGATGATGGTGATGAT** | **56** |
| **5IMM** | **6** | **F** | **CCGGCGATGGCCATGCAAGTTCAACTGGTTGAATC** | **57** |
| | | **R** | **GTGCGGCCGCAAGCTTTAGTGGTGATGGTGATGAT** | **58** |
| **5JQH** | **8** | **F** | **CCGGCGATGGCCATGCAAGTCCAACTGCAAGAATC** | **59** |
| | | **R** | **GTGCGGCCGCAAGCTTTAGTGGTGATGGTGATGAT** | **60** |
| **5NBD** | **10** | **F** | **CCGGCGATGGCCATGCAAGTCCAACTGCAAGAATC** | **61** |
| | | **R** | **GTGCGGCCGCAAGCTTTAATGATGATGATGGTGAT** | **62** |

### (2) VHH production

The recombinant *Escherichia coli* produced in (1) was inoculated in 2 mL of an LB medium containing 50 ppm sodium ampicillin and was subjected reciprocal shaking overnight at 30°C to prepare a pre-culture solution. Twenty microliters of the pre-culture solution was inoculated in 2 mL of Overnight Express^{™} Instant TB Medium, followed by culturing at 30°C for 24 hours.

### (3) SDS-PAGE and Western blotting

Cells were collected from the culture obtained in (2) by centrifugation. The obtained cells were resuspended in a PBS buffer in an equal amount to that of the culture solution subjected to the centrifugation, and Laemmli Sample Buffer (Bio-Rad Laboratories, Inc.) containing 50 mM DTT was added to the suspension to prepare a sample. The resulting sample was subjected to SDS-PAGE by the same procedure as in Example 1 (5). Western blotting was performed by the same procedure as in Example 1 (6).

### (4) Results

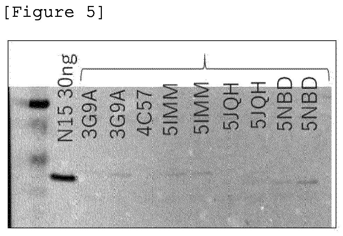

Figure 5 shows SDS-PAGE images of *Escherichia coli* strain cultures. The protein quantitative measurement resulted in that the productivity of every strain was 15 mg/L or less. Comparison with the VHH expression level (Table 3) in the Dpr9ΔsigF strain showed that the VHH productivity of the *Bacillus subtilis* was 20-fold or more that of the *Escherichia coli.*

### Example 2 VHH production by protease deficient recombinant Bacillus subtilis

VHH expression plasmids were constructed by the same procedure as in Example 1 (1) and (2) except that polynucleotides of SEQ ID NOs: 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, and 137 (respectively encoding VHHs of SEQ ID NOs: 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, and 138: referred to as VHHs 1 to 38) were used as the VHH genes. The constructed plasmids were introduced into Dpr9ΔsigF in accordance with the procedure shown in Example 1 (3). The produced recombinant *Bacillus subtilis* strains were cultured by the same procedure as in Example 1 (4) to express the VHHs.

The VHHs expressed by the recombinant *Bacillus subtilis* were quantitatively measured by ELISA. The ELISA was performed in accordance with the manual of His Tag Antibody Plate (GenScript). The supernatants of the cultures of the recombinant *Bacillus subtilis* obtained above were each diluted 20,000-fold with PBS, and 100 µL of the diluted solutions were placed in each well of His Tag Antibody Plate and were left to stand overnight at 4°C. The wells were washed with PBS containing 0.05% of Tween (registered trademark) 20 (PBST) three times. Subsequently, 100 µL of Peroxidase-conjugated AffiniPure Goat Anti-Alpaca IgG, VHH domain (Jackson ImmunoResearch 128-035-232) diluted 5,000-fold with PBST was added to each well, followed by leaving to stand at room temperature for 30 minutes. The wells were then washed with PBST three times, and 100 µL of 3,3',5,5'-tetramethylbenzidine (TMB [for ELISA], Tokyo Chemical Industry Co., Ltd.) was added to each well, followed by leaving to stand at room temperature for 20 minutes. The reaction was stopped with addition of 50 µL of TMB stop solution (SeraCare Life Sciences, Inc.), and the absorbance at 450 nm of each well was then measured. The protein concentration was calculated in accordance with the manual of Ni-NTA Agarose (FUJIFILM Wako Pure Chemical Corporation) using the absorbance of VHH 1 with known concentration in ELISA as an indicator.

Table 5 shows expression levels of VHHs 1 to 38 in the recombinant *Bacillus subtilis* strain Dpr9ΔsigF. All the VHHs 1 to 38 were produced from the recombinant *Bacillus subtilis* strain.

**[Table 5]**

| **VHH** | **SEQ ID:No** | **Expression level (q/L)** | | **VHH** | **SEQ ID:No** | **Expression level (g/L)** |
|---|---|---|---|---|---|---|
| **VHH1** | **64** | **>2** | | **VHH20** | **102** | **1.2** |
| **VHH2** | **66** | **1.7** | | **VHH21** | **104** | **>2** |
| **VHH3** | **68** | **>2** | | **VHH22** | **106** | **>2** |
| **VHH4** | **70** | **2.0** | | **VHH23** | **108** | **>2** |
| **VHH5** | **72** | **>2** | | **VHH24** | **110** | **>2** |
| **VHH6** | **74** | **>2** | | **VHH25** | **112** | **>2** |
| **VHH7** | **76** | **1.1** | | **VHH26** | **114** | **>2** |
| **VHH8** | **78** | **1.0** | | **VHH27** | **116** | **>2** |
| **VHH9** | **80** | **1.2** | | **VHH28** | **118** | **1.65** |
| **VHH10** | **82** | **>2** | | **VHH29** | **120** | **1.15** |
| **VHH11** | **84** | **>2** | | **VHH30** | **122** | **0.16** |
| **VHH12** | **86** | **1.94** | | **VHH31** | **124** | **1.74** |
| **VHH13** | **88** | **0.45** | | **VHH32** | **126** | **0.39** |
| **VHH14** | **90** | **1.67** | | **VHH33** | **128** | **0.1** |
| **VHH15** | **92** | **>2** | | **VHH34** | **130** | **0.11** |
| **VHH16** | **94** | **>2** | | **VHH35** | **132** | **0.08** |
| **VHH17** | **96** | **>2** | | **VHH36** | **134** | **>2** |
| **VHH18** | **98** | **1.04** | | **VHH37** | **136** | **0.37** |
| **VHH19** | **100** | **1.9** | | **VHH38** | **138** | **1.13** |

| | | | | | | |
|---|---|---|---|---|---|---|
| *: > 2 is 2 g/L or more, outside the upper limit of the calibration curve. | | | | | | |

While embodiments of the present invention have been described above, it should be understood that they are not intended to limit the present invention to specific embodiments described. Various other changes and modifications within the scope of the present invention are obvious to those skilled in the art. The documents and patent applications cited herein are incorporated by reference in their entirety.

## Claims

1. A method for producing a variable domain of heavy chain of heavy-chain antibody, comprising culturing a *Bacillus* bacterium into which a gene that encodes variable domain of heavy chain of heavy-chain antibody is introduced and which is deficient in an extracellular protease.

2. The method according to Claim 1, wherein the extracellular protease is at least one selected from the group consisting of extracellular proteases encoded by epr, wprA, bpr, vpr, aprE, aprX, and genes equivalent to these genes.

3. The method according to Claim 1 or 2, wherein the variable domain of heavy chain of heavy-chain antibody consists of an amino acid sequence of SEQ ID NO: 2, 4, 6, 8, 10, 12, 14, 16, 18, 20, 22, 24, 26, 28, 30, 32, 34, 36, 38, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, or 138 or an amino acid sequence having an identity of at least 85% to any of these sequences.

4. The method according to any one of Claims 1 to 3, wherein the *Bacillus* bacterium is *Bacillus subtilis* or a mutant strain thereof.

5. The method according to any one of Claims 1 to 4, wherein the *Bacillus* bacterium deficient in an extracellular protease is a *Bacillus* bacterium in which the gene that encodes the extracellular protease is deleted or inactivated.

6. A *Bacillus* bacterium into which a gene that encodes variable domain of heavy chain of heavy-chain antibody is introduced and which is deficient in an extracellular protease.

7. The *Bacillus* bacterium according to Claim 6, wherein the extracellular protease is at least one selected from the group consisting of extracellular proteases encoded by epr, wprA, bpr, vpr, aprE, aprX, and genes equivalent to these genes.

8. The *Bacillus* bacterium according to Claim 6 or 7, wherein the variable domain of heavy chain of heavy-chain antibody consists of an amino acid sequence of SEQ ID NO: 2, 4, 6, 8, 10, 12, 14, 16, 18, 20, 22, 24, 26, 28, 30, 32, 34, 36, 38, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, or 138 or an amino acid sequence having an identity of at least 85% to any of these sequences.

9. The *Bacillus* bacterium according to any one of Claims 6 to 8, which is *Bacillus subtilis* or a mutant strain thereof.

10. The *Bacillus* bacterium according to any one of Claims 6 to 9, wherein the gene that encodes the extracellular protease is deleted or inactivated.
